# EUROPEAN PATENT SPECIFICATION

(11) **EP 0 761 172 B1**
(45) Date of publication and mention of the grant of the patent: **05.06.2002**
(21) Application number: 96401802.2
(22) Date of filing: 21.08.1996
(51) Int. Cl.: A61B 17/15

(54) **Apparatus for correction of spaces between bone resection planes for knee prostheses**
Gerät zur Richtigstellung der Abstände zwischen Knochenresektionsebenen für Knieprothesen
Appareil de correction d'espaces entre plans de résection des os pour prothèses du genou

(30) Priority: 24.08.1995 FR 9510055
(43) Date of publication of application: 12.03.1997
(73) Proprietor: BENOIST GIRARD SAS, 14200 Hérouville-Saint-Clair (FR)
(72) Inventor: Baron, Lionel, 14150 Ouistreham (FR); Schifrine, Patrick, 74290 Veyrier du Lac (FR); van de Velde, Denis, 59300 Valenciennes (FR); Moncade, Nicolas, 67114 Echau (FR); Setiey, Louis, 69400 Gleize (FR); Segal, Philippe, 51350 Cormontreuil (FR); Raguet, Marc, 51000 Chalons S/Marne (FR); Vielpeau, Claude, 14200 Herouville Saint Clair (FR); Balay, Bruno, 01600 Saint Bernard (FR); Francois, Jean-Marie, 67500 Marienthal (FR); Stahl, Philippe, 59262 Sainghain en Melantois (FR); Serrault, Michel, 61100 Flers (FR); Rivat, Paul, 07130 Saint Peray (FR); Mailhe, Didier, Le Clos des Feuillantines, 34170 Castelnau Le Lez (FR); Monteleone, Vittorio, Napoli 80121 (IT); Verleyen, Jean-Marie, La Croix des Rampaux, 69520 Grigny (FR)
(74) Representative: Rinuy, Santarelli

(56) References cited:
- DE-U- 9 301 611
- FR-A- 2 648 699
- JOURNAL OF BONE AND JOINT SURGERY, vol. 65a, no. 3, March 1983, BOSTON,MASSACHUSETTS,USA, pages 293-309, XP002001527 N.S.EFTEKHAR: "Total Knee-Replacement Arthroplasty"

## Description

The present invention concerns space correction apparatus for knee prostheses.

A conventional knee prosthesis generally includes a tibial member implanted in a resection plane of the proximal upper part of the tibia, an intermediate meniscus and a femoral member with two condyles requiring, for implanting it in the distal lower part of the femur, three resection planes of which one corresponds to the extension position and the other two to the flexion position, and one, or even two, chamfers in planes at approximately 45° on either side of the resection plane of the femur corresponding to the extension position.

To implant a knee prosthesis of this kind the upper proximal part of the tibia and the lower distal part of the femur must be resectioned so that there is the same distance between the resection planes of the two bones, both in the extended position of the leg (corresponding to the standing position of the patient) and in the flexed position of the leg (corresponding to the seated position).

To meet this condition and to enable the implantation of the prosthesis it is necessary to measure the exact distance between the cutting planes of the tibia and of the femur in the extended position, selecting a given tension balanced ligamentary and to transfer this distance to the flexed position to determine the second and third resection planes of the femur, which are in practice very nearly orthogonal to the first resection plane of the femur, and to make at least one and preferably two chamfers in planes each inclined at an angle of approximately 45° to said resection planes of the distal end of the femur, as defined hereinabove.

The present invention consists in apparatus enabling the surgeon to adjust the space between the proximal resection plane of the tibia and the distal resection plane of the femur in the extended position and to determine the second cutting (or resection) plane or planes of the femur in the flexed position and where applicable the cutting planes of the chamfers, complying with said space as previously determined.

The space correction apparatus for knee prostheses in accordance with the invention is characterised in that it comprises a gantry and two pairs of skids, disposed one above the other, and all parallel to each other, and extending in a plane perpendicular to the gantry to form rear parts and short of the front face of the gantry to form front parts, wherein:
(i) the front parts and rear parts of the lower pair of skids are fastened together and can be moved simultaneously relative to the gantry;
(ii) the front parts and rear parts of the top pair of the skids are not fastened together;
(iii) the front parts of the top pair of skids can be moved individually relative to the gantry; and
(iv) the rear parts of the top pair of skids are both fastened to the gantry.

The apparatus includes a sighting turret fixed to the gantry to pivot about an axis and preferably including a device for measuring the size of the femoral member and it can receive a removable anterior-posterior cutting guide that can be fixed to the gantry at a particular position. Document FR-A-2648699 discloses an apparatus with the features specified in the preamble of claim 1.

The apparatus of the invention is used in surgery as follows.

After resection of the proximal end of the tibia and the distal end of the femur in the extended position, the surgeon introduces the rear (extension) parts of the two pairs of skids of the apparatus, which have previously been placed in contact with each other, between the two resection planes and moves the two pairs of skids apart until they abut on said planes, to obtain the separation to be transferred to the flexed position.

The surgeon then balances the lateral ligamentary tension, in this position, in which the rear part of the top pair of skids is in contact with the resectioned location of the distal condyles of the femur, and verifies the hip-knee-ankle (HKA) alignment using a longitudinal rod threaded into the sighting turret, i.e. the alignment of the rod with the femoral-tibial anatomical axis of the patient, which runs from the hip (the centre of the head of the femur) to the centre of the ankle.

After removing the apparatus and fixing the anterior-posterior cutting guide to the gantry the surgeon introduces the front (flexion) part of the two pairs of skids of the apparatus between the tibia and the fibia in the flexed position, transfers the spacing obtained in the extended position and then, if necessary, individually moves the front part of one or other of the skids of the top pair to turn the femur and therefore tension the ligaments, which stabilises and recentres the ball joint.

The position of the cutting guide on the gantry and its shape determine the anterior and posterior cutting planes of the femur in the flexed position. The guide is then fixed to the resectioned distal part of the femur by any appropriate means; the guide is separated from the apparatus and the surgeon can then saw the anterior and posterior cutting planes of the femur, which are practically orthogonal to the resectioned distal part of the femur, and the chamfer or the two chamfers required for implanting the femoral member of the prosthesis.

Choosing the size of the femoral member is facilitated by the size measuring device.

The invention that is the subject matter of the present patent application will now be described in one particular embodiment in which:
- figure 1 is a perspective view of the apparatus fitted with a sighting turret with a device for measuring the size of the femoral member of the prosthesis;
- figures 2, 3, 4 and 5 are respectively side, front, top and perspective views of a first member of the apparatus from figure 1 called the spreader;
- figures 6, 7, 8, 9 and 10 are respectively a front view, a section view on the line AA in figure 8, a lateral view, a top view and a perspective view of a second member of the apparatus from figure 1 called the gantry;
- figures 11, 12, 13 and 14 are respectively a front view in section on the line BB in figure 12, a lateral view, a top view and a perspective view of the front part of one of the skids of the top pair of skids;
- figures 15, 16, 17 and 18 are respectively a front view in section on the line CC in figure 16, a lateral view, a top view and a perspective view of a sighting turret support with a device for measuring the size of the femoral member of the prosthesis shown in figure 1;
- figures 19 through 22 are respectively a side view, a front view, a top view and a perspective view of a sighting turret including a device for measuring the size of the femoral member of the knee prosthesis;
- figures 23 and 24 respectively show central operating screws of the lower pair of skids and the side of the front part only of one of the skids of the top pair of skids in the apparatus of the invention;
- figures 25 through 28 are respectively a front view, a side view, a rear view and a perspective view of a counter-plate used with the apparatus from figure 1;
- finally, figures 29 through 31 are respectively perspective views laid flat and standing up and a sectional view on the line EE in figure 29 of an anterior-posterior cutting guide that can be fixed to the apparatus of the invention.

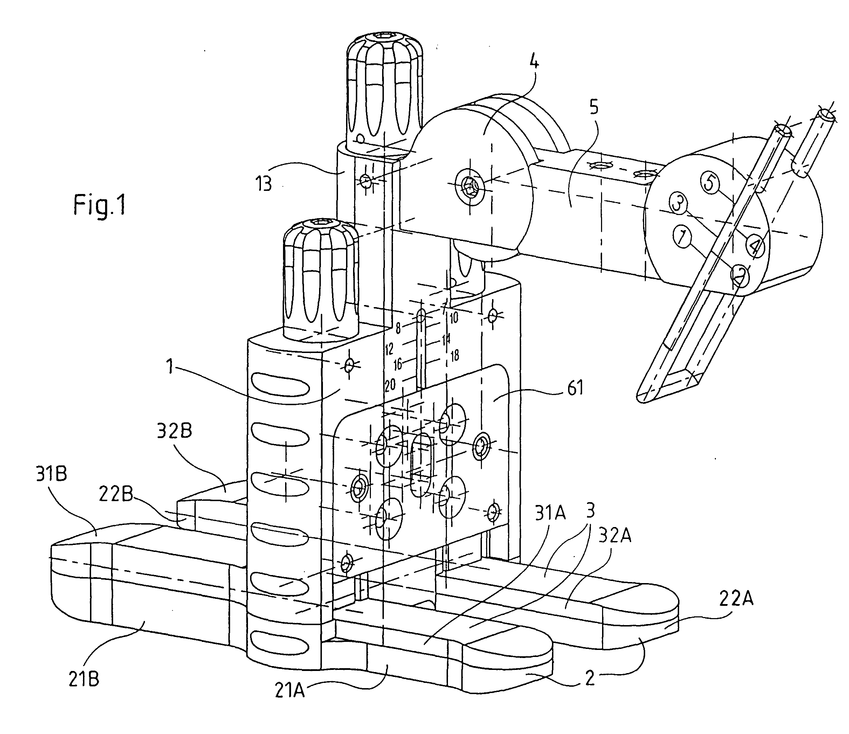

Referring to figure 1, the apparatus of the invention includes a gantry (1) and two pairs (2 and 3) of skids disposed one above the other and lying in a plane perpendicular to that of the gantry.

The skids of the two pairs are parallel and each includes a relatively thicker rear part (21B, 22B and 31B, 32B) to the rear of the plane of the gantry, and a relatively thinner front part (21A, 22A and 31A, 32A) to the front of the plane of the gantry, as shown in figure 1.

The gantry (1) includes three parallel bores, the vertical axes of which are in a common plane which defines the plane of the gantry.

Each of these bores receives an operating screw with a knurled knob at the top, the central bore also including a support (4) for a sighting turret (5) fitted with a device for measuring the size of the femoral member and mounted on a tubular extension (13).

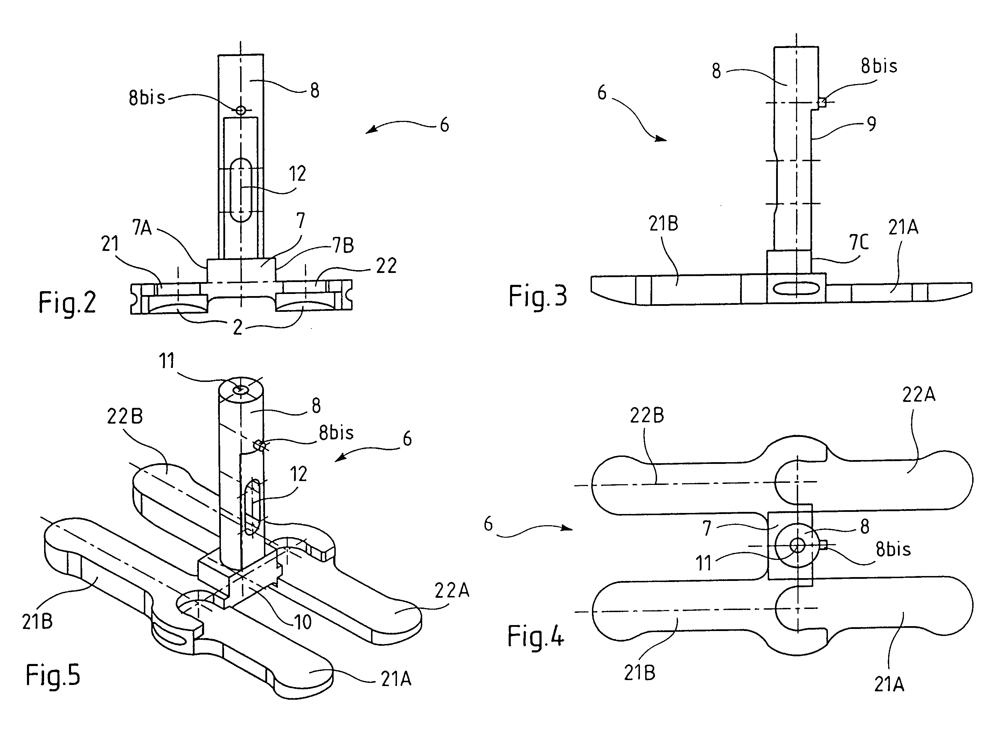

As shown by a part called the spreader (6), shown in figures 2 through 5, the bottom pair (2) of skids (21, 22) are attached by a spacer (7) to a central pillar (8).

The spacer (7) has two lateral faces (7A and 7B) that mate with the gantry (1).

The central pillar (8) has a circular cross-section with two parallel flats. One flat (9) is in the same plane as the front face (7C) of the spacer and the step (10) due to the difference in thickness between the front parts (21A and 22A) and the rear parts (21B and 22B) of the skids (21 and 22).

The central pillar (8) also includes a screwthreaded bore (11) along the axis of the pillar, starting in the upper part, opposite the skids, and opening into an oblong hole (12) through the pillar, between the two flats (see figure 2).

Finally, said pillar (8) has a stud (8bis), shown in figures 2 through 5, designed to cooperate with a vertical groove (27bis) on the gantry (1) to define the relative displacement of these members and to serve as a cursor moving along a graduation indicating the thickness of the meniscus of the knee prosthesis.

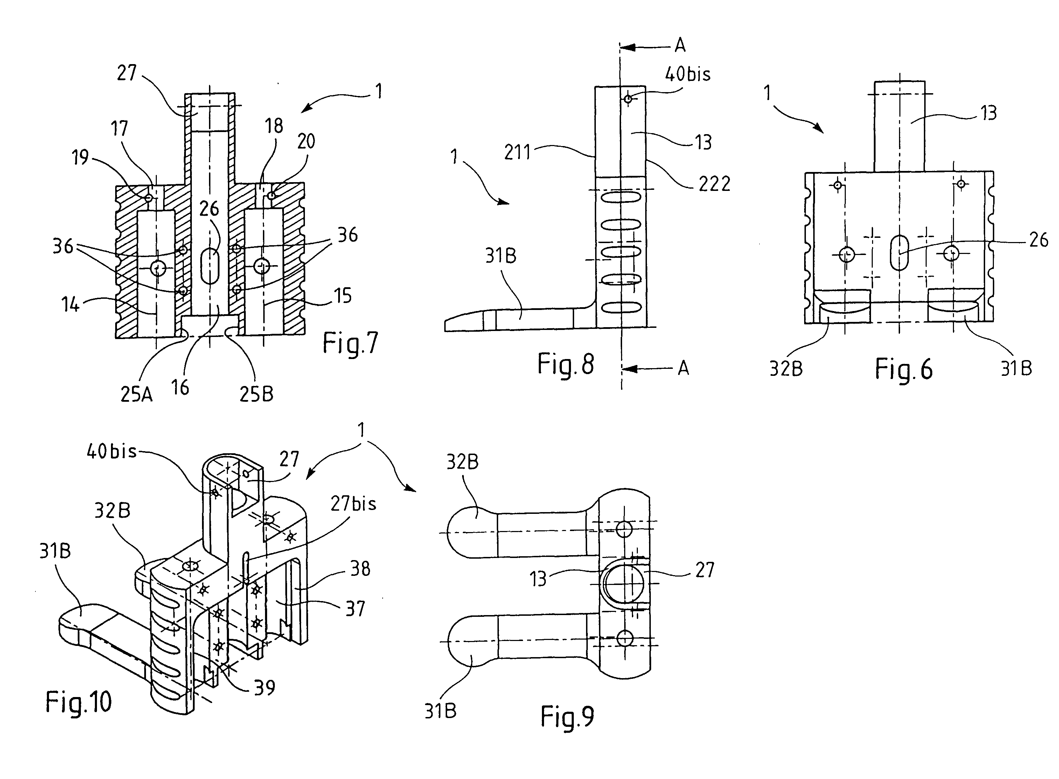

The gantry (1) of the apparatus of the invention will now be described with the aid of figures 6 through 10.

Said gantry, which has an inverted U-shape, is attached to a central tubular extension (13) and the rear parts (31B and 32B) of the top pair (3) of skids (31 and 32).

As shown in the section on AA, in the plane defined by their axes, the body (1) includes three parallel cylindrical bores: the two lateral bores (14 and 15) are designed to receive the operating pillars of the front parts of the skids of the top pair and the central bore (16) is designed to receive the central pillar (8) of the spreader (6).

The two lateral bores (14 and 15) are open at their bottom end and are extended at their top end by a smaller diameter screwthreaded bore (17 and 18) receiving a lateral operating screw.

A lateral pin (19 and 20) passes at right angles through the bores (17 and 18) and gives onto the lateral walls (211 and 222) of the gantry.

The central bore (16) has mating faces (25A and 25B) in its bottom part that cooperate with the mating faces (7A and 7B) of the spacer (7) of the spreader (6), an oblong hole (26) in its middle part corresponding to the oblong hole (12) in the spreader (6) and a central tubular extension (13) extending it in the upper part.

The central tubular extension (13) includes a notch (27) with a pin (40bis) for receiving and immobilising the support (4) of the sighting turret (5) with the device for measuring the size of the femoral member, as shown in figure 1.

Finally, the gantry (1) includes on its front face means for positioning a counter-plate and cutting guide which may include, for example, screwthreaded bores (36) and a window (37) delimited by vertical lateral walls (38 and 39).

The front face of said gantry (1) also includes a vertical groove (27bis) shown in figure 10 cooperating with the stud (8bis) on the pillar (8) as previously indicated.

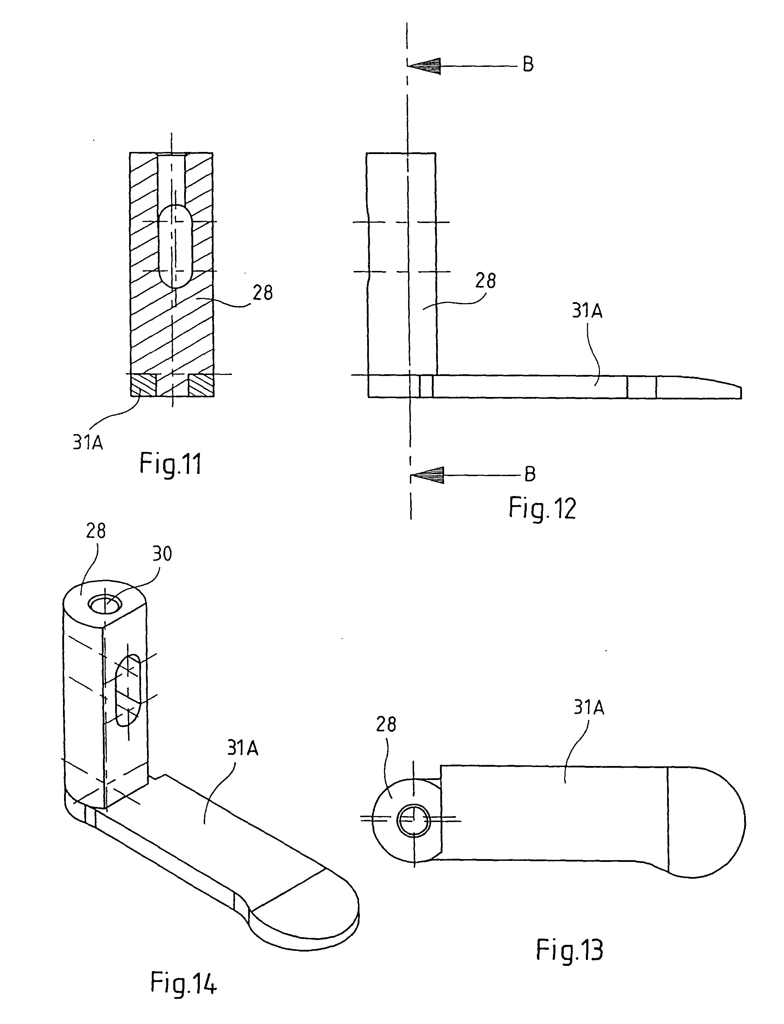

As shown in figures 11 through 14, each front part (31A or 32A) of the top pair (3) of skids (31 and 32) is attached to an operating pillar (28 or 29) through which there extends an axial screwthreaded bore (30 or 33).

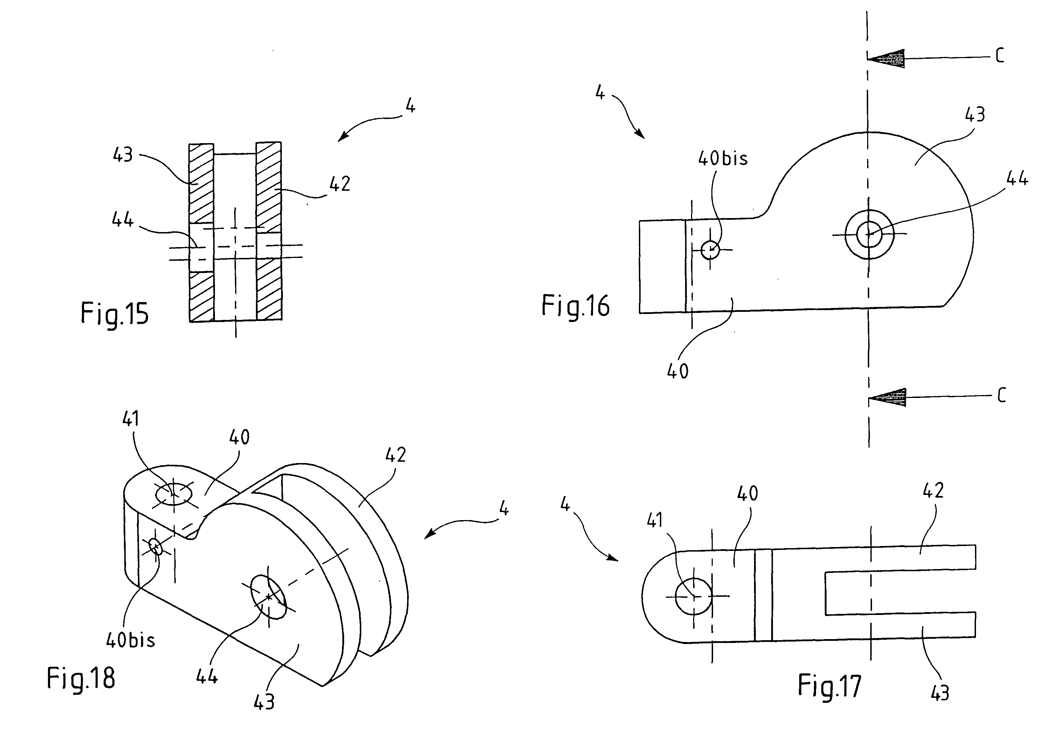

The sighting turret support (4) with the device for measuring the size of the femoral member is shown in figures 15 through 18; it comprises a seat (40) including a pin (40bis) and a screwthreaded bore (41) and attached to two lugs (42, 43) incorporating a bore (44) the axis of which is perpendicular to that of the bore (41); the bore (41) is designed to receive the central operating screw and the bore (44) is designed to receive the spindle of a sighting turret (5) with a device for measuring the size of the femoral member.

The support (4) cooperates with the notch (27) on the central tubular extension (13) to be oriented in a direction parallel to the axis of the two pairs of skids of the apparatus and the two 90° sector-shape lugs (42, 43) receive between them the tail (45) of a sighting turret (5) with the device described above.

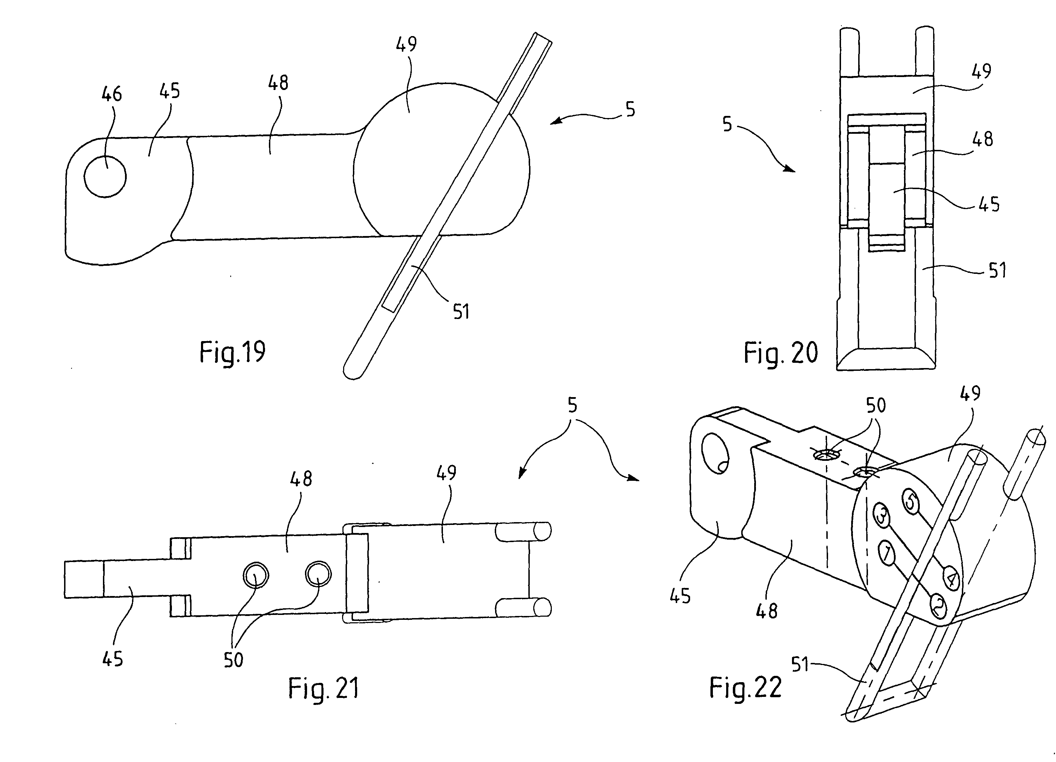

The sighting turret (5) fitted with a device for measuring the size of the femoral member, is shown in figures 19 through 22. The sighting turret (5) includes a tail (45) incorporating a bore (46) and attached to a sleeve (48) including at least one bore (50) the axis of which is perpendicular to that of the bore (46).

The tail (45) of the sighting turret (5) can be inserted between the lugs (42, 43) of the support (4) to align their respective bores (44 and 46) and to receive a pivot pin.

The sighting turret (5) can turn approximately 90° about this pin and a long rigid rod, in the order of 1 metre to 1.5 metres long, can be threaded into one of the bores (50) and immobilised to provide a visual representation of a direction parallel to the femoral-tibial anatomical axis of the patient and to enable the surgeon to verify the HKA alignment when the rear parts of the skids of the apparatus are inserted between the tibia and the fibia of the patient in the extended position of the latter's leg.

The sleeve (48) of the sighting turret (5) ends in a cylindrical member (49).

Said cylindrical member (49) includes, for measuring the size of the femoral member of the prosthesis, an inverted U-shape rigid pin (51) sliding in two parallel oblique grooves on the opposite faces of the cylindrical member (49) and carrying a marker moving along a graduation on one of those faces, as shown in figure 22.

When said device (5) is in place on the support (4) in the position shown in figure 1, the resectioned end of the femur being disposed on the front parts (31A and 32A) of the skids, the surgeon can slide the pin (51) downwards until it contacts the anterior cortex extending the resection plane of the femur. The surgeon can then determine the size of the femoral member of the prosthesis by reading off the position at which the marker has stopped on the graduation of the cylindrical member (see figure 22).

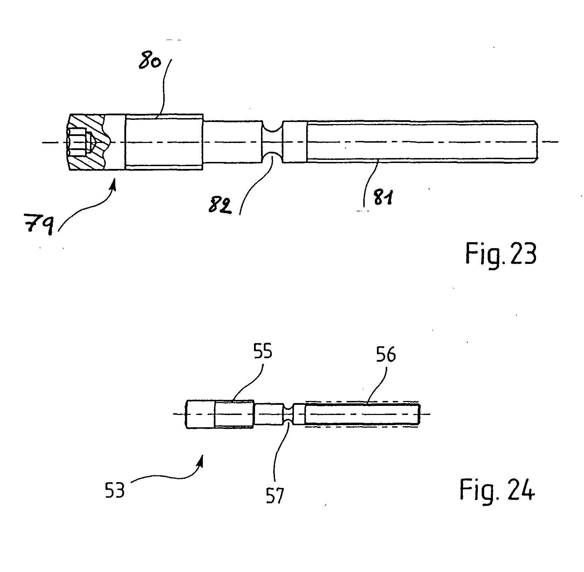

The central operating screw (79) shown in figure 23 has a head attached to a central knurled knob and the body of the screw has, starting from the head, two successive screwthreads (80 and 81), of which the latter has a smaller diameter than the former, separated by a constriction (52).

The end screwthread (81) cooperates with the screwthreaded bore (11) in the central pillar (8) of the spreader (6), the constriction (82) cooperates with the pin (40bis) of the tubular extension (13) and the sighting turret support (4) and the screwthread (80) is attached to the central knurled button.

The lateral operating screws (53, 54) are identical. One of them is shown in figure 24; it has a head attached to a lateral knurled knob and the body of the screw has, starting from the head, two successive screwthreads (55 and 56), of which the latter has a smaller diameter than the former, separated by a constriction (57).

The constriction (57) cooperates with the pin (19 or 20) of the body (1), the screwthread (56) cooperates with the screwthread (30) of the pillar (29 - 32) and the screwthread (55) is attached to a lateral knurled knob.

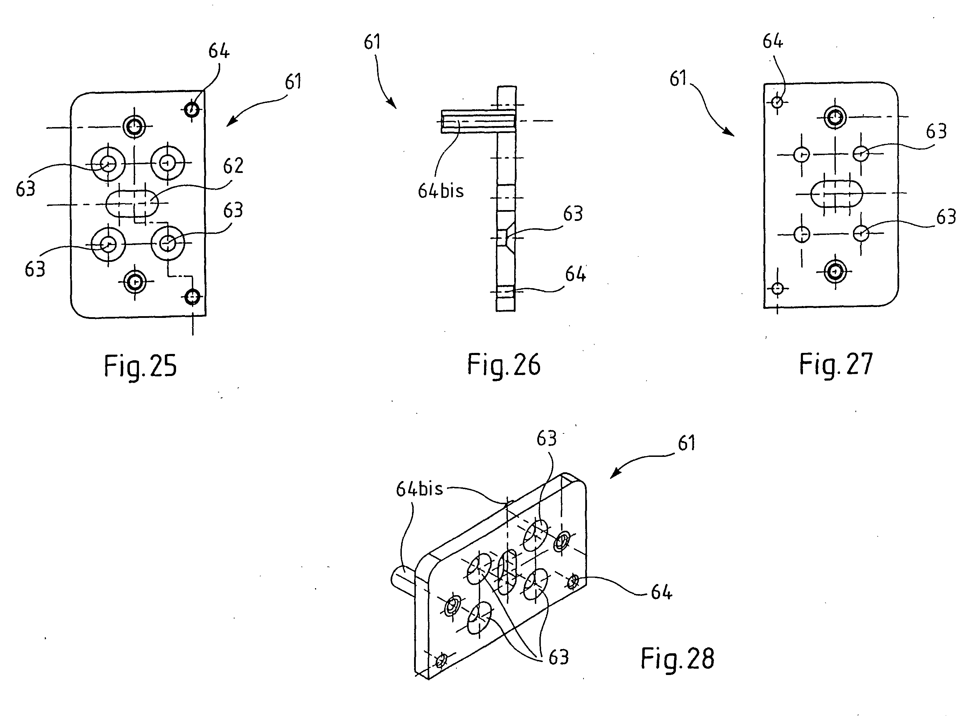

A counter-plate (61) shown in figures 25 through 28 and including an oblong hole (62) of similar dimensions to the oblong holes (12 and 26), perforations (63, 64) and tenons (64bis) with holes through them enabling the placing of two pins perpendicularly to the distal femoral cutting plane has dimensions enabling it to fit perfectly between the walls (38 and 39) of the window (37) in the gantry (1) and to be screwed to the latter at (36).

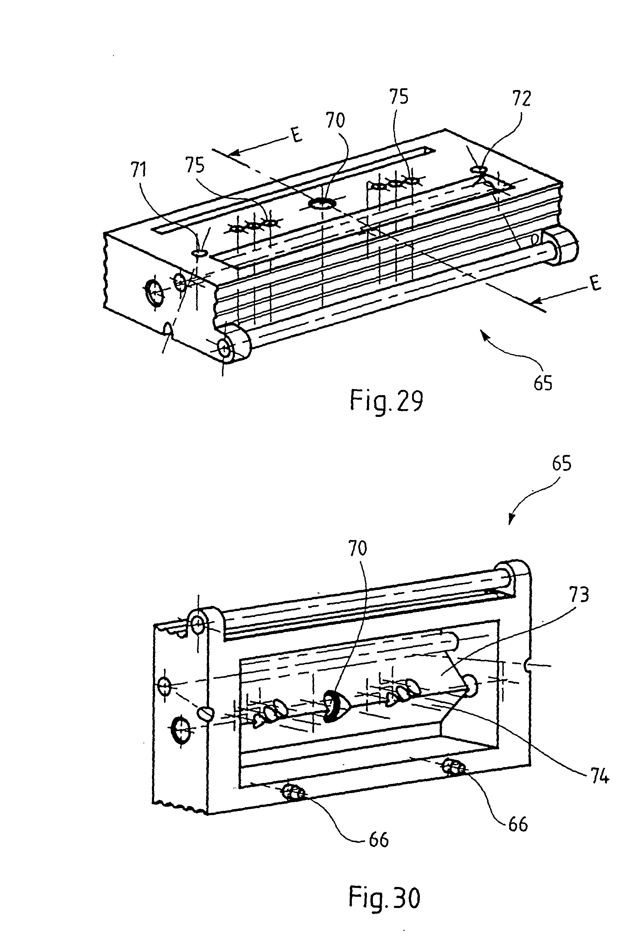

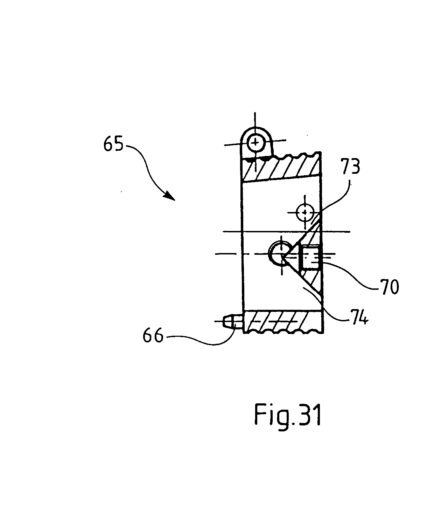

Finally, the apparatus of the invention may be fitted with a cutting guide (65) shown in figures 29 through 31.

This anterior-posterior cutting guide (65) is in the form of a block with a trapezoidal cross-section the top and bottom surfaces of which serve to support a surgical saw to define the anterior and posterior cutting planes of the femur in the flexed position of the leg of the patient.

The cutting guide (65) has locating means cooperating with the gantry (1): these locating means may comprise projections (66) cooperating with the perforations (64) in the counter-plate (61).

The cutting guide (65) may also include one or more screwthreaded bores (70) enabling it to be fixed to the gantry (1), in a mated position, using an appropriate tool.

It further includes a plurality of perpendicular holes (75) corresponding to those in the tenons (64bis) enabling modification of the position of the cutting template (65) and one or preferably two pairs of bores oriented at approximately 45° and converging from the face including the locating means, as indicated by the reference numbers (71, 72).

It can also have two planes (73 and 74) inclined at approximately 45° and designed to guide a surgical saw for chamfering the resection planes of the femur.

In the apparatus of the invention the bottom skids (21, 22) are mobile simultaneously and the front parts (31A, 32A) of the top skids (31, 32) are mobile individually in translation in the direction of the axis of the operating screws (49, 53 and 54).

Rotating one of the lateral operating screws (53, 54) when the cutting guide (65) is in contact with the femur in the flexion position enables the ball joint to be stabilised and centred before making the cuts corresponding to that position.

Accordingly, the apparatus enables the surgeon to verify that the separation of the resectioned parts of the femur and the tibia is the same in the extended position and in the flexed position and to check that the ligamentary tension is correct and that the ball joint is centred before carrying out the distal resection of the femur in the flexed position.

## Claims

1. Space correction apparatus for knee prostheses comprising a gantry (1) and two pairs (2, 3) of skids (21, 22, 31, 32), disposed one above the other and parallel to each other, the skids extending in a plane perpendicular to the gantry (1) **characterised in that** said skids have rear parts (21B, 22B, 31B, 32B) extending to the rear of the gantry (1) and front parts (21A, 22A, 31A, 32A) extending to the front of the gantry (1), wherein:
(i) the front parts (21A, 22A) and rear parts (21B, 22B) of the lower pair (2) of skids (21, 22) are fastened together and can be moved simultaneously relative to the gantry (1);
(ii) the front parts (31A, 32A) and rear parts (31B, 32B) of the top pair (3) of skids (31, 32) are not fastened together;
(iii) the front parts (31A, 32A) of the top pair (3) of skids (31, 32) can be moved individually relative to the gantry (1); and
(iv) the rear parts (31B, 32B) of the top pair (3) of skids (31, 32) are both fastened to the gantry (1).

2. Apparatus according to claim 1 **characterised in that** the front and rear parts of the bottom pair of skids (21, 22) can be moved simultaneously by turning a central operating screw (79) mounted on the gantry (1), the axis of which is perpendicular to the plane defined by said skids (21, 22), thereby allowing said skids to move in translation in the direction of said axis.

3. Apparatus according to claim 1 or claim 2 **characterised in that** the front part (31A, 32A) of each of the skids (31, 32) of the top pair (3) can be moved individually by turning a lateral operating screw (53, 54) mounted on the gantry (1), the axis of which is perpendicular to the plane defined by said skids, thereby allowing said skids to move in translation in the direction of said axis.

4. Apparatus according to claim 3 **characterised in that** the gantry (1) includes three screws (79, 53, 54) the axes of which are parallel to each other and perpendicular to the plane defined by the pair of skids (2 3), a central screw (79) moving the bottom pair (2) of skids (21, 22) and the two lateral screws (53, 54) each moving the front part (31A, 32A) of the top pair (3) of skids (31, 32).

5. Apparatus according to claim 4 **characterised in that** it includes a central pillar (8) 'attached to the bottom pair (2) of skids (21, 22) and a gantry (1) attached to the rear parts (31B, 32B) of the top pair (3) of skids, said gantry (1) having a central bore (16) receiving the central pillar (8) and two lateral bores (14, 15) through which the three screws (79,53,54) pass.

6. Apparatus according to any one of claims 1 to 5 **characterised in that** it includes a sighting turret (5) optionally including a device for measuring the size of the femoral member, adapted to provide a visual indication of the direction of the femoral-tibial axis in the extended position and disposed at the centre of the gantry (1), said sighting turret optionally being able to pivot within a range of 90° in a plane orthogonal to that of the gantry (1) and to that of the skids (21, 22, 31, 32).

7. Apparatus according to any one of claims 1 to 5 **characterised in that** it includes a removable anterior-posterior cutting guide (65) in the form of a trapezoidal cross-section block having locating means (66) cooperating with locating means (64) of a counter-plate (61) for fixing it to the apparatus at a particular position, means (71, 72) enabling it to be fixed to a resectioned plane surface of the femur and means (73, 74) for defining at least one plane to be cut by a surgical saw.

8. Apparatus according to claim 7 **characterised in that** the cutting guide (65) is located on the gantry (1) by engagement of a projection (66) in a corresponding perforation (64) and/or by means of a screw (70).

9. Apparatus according to claim 7 or claim 8 **characterised in that** the means for fixing the cutting guide to a resectioned plane surface of the femur comprise one or two pairs of bores (71, 72) preferably inclined at approximately 45° to a plane face of the cutting guide.

10. Apparatus according to any one of claims 7 to 9 **characterised in that** the means on the cutting guide for defining at least one cutting plane are firstly its thickness, its bottom surface and its top surface serving as cutting planes for a surgical saw, and secondly two planes (73, 74) inclined at approximately 45° enabling the insertion of a saw to make chamfers at the 90° resectioned distal end of the femur.

11. Apparatus according to any one of claims 1 to 6 **characterised in that** the rear parts (21B, 22B) of the pair (2) of bottom skids (21, 22) are thicker than the front parts (21A, 21B) of the same pair.

12. Apparatus according to any one of claims 1 to 7 **characterised in that** the rear parts (31B, 32B) of the pair (3) of top skids (31, 32) are thicker than the front parts (31A, 31B) of the same pair.

## Patentansprüche

1. Abstandskorrekturvorrichtung für Knieprothesen umfassend einen Montagerahmen (1) und zwei Paare (2, 3) von Kufen (21, 22, 31, 32), die übereinander angeordnet und untereinander parallel sind, wobei sich die Kufen in einer zum Montagerahmen (1) senkrechten Ebene erstrecken, **dadurch gekennzeichnet, dass** die besagten Kufen Hinterteile (21B, 22B, 31B, 32B) aufweisen, die sich zur Rückseite des Montagerahmens (1) erstrecken, und Vorderteile (21A, 22A, 31A, 32A) aufweisen, die sich zur Vorderseite des Montagerahmens (1) erstrecken, wobei:
(i) die Vorderteile (21A, 22A) und Hinterteile (21B, 22B) des unteren Paars (2) der Kufen (21, 22) fest miteinander verbunden und in bezug zum Montagerahmen (1) gleichzeitig bewegt werden können;
(ii) die Vorderteile (31A, 32A) und Hinterteile (31B, 32B) des oberen Paars (3) der Kufen (31, 32) nicht fest miteinander verbunden sind;
(iii) die Vorderteile (31A, 32A) des oberen Paars (3) der Kufen (31, 32) in bezug zum Montagerahmen (1) einzeln bewegt werden können; und
(iv) die Hinterteile (31B, 32B) des oberen Paars (3) der Kufen (31, 32) beide fest mit dem Montagerahmen (1) verbunden sind.

2. Vorrichtung nach Anspruch 1, **dadurch gekennzeichnet, dass** die Vorder- und Hinterteile des unteren Paars der Kufen (21, 22) durch Drehung einer mittleren Bedienungsschraube (79), die auf dem Montagerahmen (1) angebracht ist, deren Achse zu der von den besagten Kufen (21, 22) definierten Ebene senkrecht ist, gleichzeitig bewegt werden können, wodurch sich die Kufen in einer Translationsbewegung in Richtung der besagten Achse bewegen können.

3. Vorrichtung nach Anspruch 1 oder 2, **dadurch gekennzeichnet, dass** das Vorderteil (31A, 32A) von jeder der Kufen (31, 32) des oberen Paars (3) durch Drehung einer seitlichen Bedienungsschraube (53, 54), die auf dem Montagerahmen (1) angebracht ist, deren Achse zu der von den besagten Kufen definierten Ebene senkrecht ist, einzeln bewegt werden können, wodurch sich die besagten Kufen in einer Translationsbewegung in Richtung dieser Achse bewegen können.

4. Vorrichtung nach Anspruch 3, **dadurch gekennzeichnet, dass** der Montagerahmen (1) drei Schrauben (79, 53, 54) aufweist, deren Achsen parallel zueinander und zu der von dem Paar der Kufen (2, 3) definierten Ebene senkrecht sind, eine mittlere Schraube (79), welche das untere Paar (2) der Kufen (21, 22) bewegt, und die beiden seitlichen Schrauben (53, 54), welche jeweils das Vorderteil (31 A, 32A) des oberen Paars (3) der Kufen (31, 32) bewegen.

5. Vorrichtung nach Anspruch 4, **dadurch gekennzeichnet, dass** sie eine fest mit dem unteren Paar (2) der Kufen (21, 22) verbundene Mittelsäule (8) und einen fest mit den Hinterteilen (31B, 32B) des oberen Paars (3) der Kufen verbundenen Montagerahmen (1) aufweist, wobei der besagte Montagerahmen (1) eine die Mittelsäule (8) aufnehmende mittlere Bohrung (16) und zwei seitliche Bohrungen (14, 15) aufweist, durch welche die drei Schrauben (79, 53, 54) hindurchtreten.

6. Vorrichtung nach einem der Ansprüche 1 bis 5, **dadurch gekennzeichnet, dass** sie eine Visiereinrichtung (5) aufweist, die eine Vorrichtung zum Messen der Größe des femoralen Elements aufweisen kann und zum Sichtbarmachen der Richtung der femoro-tibialen Achse in der Strecklage vorgesehen ist und in der Mitte des Montagerahmens (1) angeordnet ist, wobei die besagte Visiereinrichtung gegebenenfalls in der Ebene, welche zu derjenigen des Montagerahmens (1) und zu derjenigen der Kufen (21, 22, 31, 32) senkrecht ist, in einem Sektor von 90° verschwenkbar ist.

7. Vorrichtung nach einem der Ansprüche 1 bis 5, **dadurch gekennzeichnet, dass** sie eine entfernbare antero-posteriore Schneidführung (65), die eine Blockform mit trapezförmigem Querschnitt und Positioniereinrichtungen (66) besitzt, welche mit Positioniereinrichtungen (64) einer Gegenplatte (61) zusammenwirken, um sie auf der Vorrichtung in einer bestimmten Position zu befestigen, Einrichtungen (71, 72), die ihre Befestigung auf einer resezierten ebenen Oberfläche des Femur ermöglichen, und Einrichtungen (73, 74) zur Festlegung mindestens einer Ebene, die durch eine chirurgische Säge zu schneiden ist, aufweist.

8. Vorrichtung nach Anspruch 7, **dadurch gekennzeichnet, dass** die Schneidführung (65) durch Eingriff eines Vorsprungs (66) in einer entsprechenden Perforation (64) und/oder durch Verschraubung (70) auf dem Montagerahmen (1) positioniert ist.

9. Vorrichtung nach Anspruch 7 oder 8, **dadurch gekennzeichnet, dass** die Einrichtungen zum Fixieren der Schneidführung auf einer resezierten ebenen Oberfläche des Femur ein oder zwei Paare von Bohrungen (71, 72) umfassen, die vorzugsweise unter einem Winkel von ungefähr 45° gegenüber einer ebenen Fläche der Schneidführung geneigt sind.

10. Vorrichtung nach einem der Ansprüche 7 bis 9, **dadurch gekennzeichnet, dass** die Einrichtungen auf der Schneidführung zur Festlegung mindestens einer Schnittebene einerseits ihre Dicke, ihre Unterseite und ihre Oberseite sind, die als Schnittebenen für eine chirurgische Säge dienen, und andererseits zwei unter einem Winkel von ungefähr 45° geneigte Ebenen (73, 74) sind, welche das Einführen einer Säge zum Herstellen von Abschrägungen an dem im rechten Winkel resezierten distalen Ende des Femur ermöglichen.

11. Vorrichtung nach einem der Ansprüche 1 bis 6, **dadurch gekennzeichnet, dass** die Hinterteile (21B, 22B) des Paars (2) der unteren Kufen (21, 22) dicker als die Vorderteile (21A, 21B) desselben Paars sind.

12. Vorrichtung nach einem der Ansprüche 1 bis 7, **dadurch gekennzeichnet, dass** die Hinterteile (31B, 32B) des Paars (3) der oberen Kufen (31, 32) dicker als die Vorderteile (31A, 31B) desselben Paars sind.

## Revendications

1. Appareil de correction d'espaces pour des prothèses du genou, comportant un portique (1) et deux paires (2, 3) de patins (21, 22, 31, 32), disposées l'une au-dessus de l'autre et parallèles entre elles, les patins s'étendant dans un plan perpendiculaire au portique (1), **caractérisé en ce que** lesdits patins comportent des parties arrière (21B, 22B, 31B, 32B) s'étendant vers l'arrière du portique (1) et des parties avant (21A, 22A, 31A, 32A) s'étendant vers l'avant du portique (1), dans lequel :
(i) les parties avant (21A, 22A) et les parties arrière (21B, 22B) de la paire inférieure (2) de patins (21, 22) sont fixées entre elles et peuvent être déplacées simultanément par rapport au portique (1) ;
(ii) les parties avant (31A, 32A) et les parties arrière (31B, 32B) de la paire supérieure (3) de patins (31, 32) ne sont pas fixées entre elles ;
(iii) les parties avant (31A, 32A) de la paire supérieure (3) de patins (31, 32) peuvent être déplacées individuellement par rapport au portique (1) ; et
(iv) les parties arrière (31B, 32B) de la paire supérieure (3) de patins (31, 32) sont fixées toutes deux au portique (1).

2. Appareil selon la revendication 1, **caractérisé en ce que** les parties avant et arrière de la paire inférieure de patins (21, 22) peuvent être déplacées simultanément en tournant une vis opératoire centrale (79) montée sur le portique (1), dont l'axe est perpendiculaire au plan défini par lesdits patins (21, 22), permettant ainsi auxdits patins de se déplacer en translation dans la direction dudit axe.

3. Appareil selon la revendication 1 ou la revendication 2, **caractérisé en ce que** la partie avant (31A, 32A) de chacun des patins (31, 32) de la paire supérieure (3) peut être déplacée individuellement en faisant tourner une vis opératoire latérale (53, 54) montée sur le portique (1), dont l'axe est perpendiculaire au plan défini par lesdits patins, permettant ainsi auxdits patins de se déplacer en translation dans la direction dudit axe.

4. Appareil selon la revendication 3, **caractérisé en ce que** le portique (1) comporte trois vis (79, 53, 54) dont les axes sont parallèles entre eux et perpendiculaires au plan défini par la paire de patins (2, 3), une vis centrale (79) déplaçant la paire inférieure (2) de patins (21, 22) et les deux vis latérales (53, 54) déplaçant chacune la partie avant (31A, 32A) de la paire supérieure (3) de patins (31, 32).

5. Appareil selon la revendication 4, **caractérisé en ce qu'**il comporte un pilier central (8) relié à la paire inférieure (2) de patins (21, 22) et un portique (1) relié aux parties arrière (31B, 32B) de la paire supérieure (3) de patins, ledit portique (1) ayant un alésage central (16) recevant le pilier central (8) et deux alésages latéraux (14, 15) à travers lesquels les trois vis (79, 53, 54) passent.

6. Appareil selon l'une quelconque des revendications 1 à 5, **caractérisé en ce qu'**il comporte une tourelle de visée (5) comprenant facultativement un dispositif destiné à mesurer la dimension de l'élément fémoral, conçue pour fournir une indication visuelle de la direction de l'axe fémoral-tibial dans la position d'extension et disposée au centre du portique (1), ladite tourelle de visée pouvant facultativement pivoter dans une plage de 90° dans un plan orthogonal à celui du portique (1) et à celui des patins (21, 22, 31, 32).

7. Appareil selon l'une quelconque des revendications 1 à 5, **caractérisé en ce qu'**il comporte un guide de coupe amovible antérieur-postérieur (65) sous la forme d'un bloc de section transversale trapézoïdale ayant des moyens de positionnement (66) coopérant avec des moyens de positionnement (64) d'une contre-plaque (61) pour sa fixation à l'appareil dans une position particulière, des moyens (71, 72) lui permettant d'être fixé à une surface d'un plan de résection du fémur et des moyens (73, 74) pour définir au moins un plan devant être coupé par une scie chirurgicale.

8. Appareil selon la revendication 7, **caractérisé en ce que** le guide de coupe (65) est positionné sur le portique (1) par l'engagement d'une saillie (66) dans une perforation correspondante (64) et/ou au moyen d'une vis (70).

9. Appareil selon la revendication 7 ou la revendication 8, **caractérisé en ce que** les moyens pour fixer le guide de coupe à une surface d'un plan de résection du fémur comprennent une ou deux paires d'alésages (71, 72) avantageusement inclinés d'environ 45° par rapport à une face plane du guide de coupe.

10. Appareil selon l'une quelconque des revendications 7 à 9, **caractérisé en ce que** les moyens situés sur le guide de coupe pour définir au moins un plan de coupe sont premièrement son épaisseur, sa surface inférieure et sa surface supérieure servant de plans de coupe pour une scie chirurgicale et, secondement, deux plans (73, 74) inclinés à environ 45°, permettant l'introduction d'une scie pour réaliser des chanfreins à l'extrémité distale réséquée à 90° du fémur.

11. Appareil selon l'une quelconque des revendications 1 à 6, **caractérisé en ce que** les parties arrière (21B, 22B) de la paire (2) de patins inférieurs (21, 22) sont plus épaisses que les parties avant (21A, 21B) de la même paire.

12. Appareil selon l'une quelconque des revendications 1 à 7, **caractérisé en ce que** les parties arrière (31B, 32B) de la paire (3) de patins supérieurs (31, 32) sont plus épaisses que les parties avant (31A, 31B) de la même paire.
